# EUROPÄISCHE PATENTSCHRIFT

(11) **EP 2 353 519 B1**
(45) Veröffentlichungstag und Bekanntmachung des Hinweises auf die Patenterteilung: **08.11.2017**
(21) Anmeldenummer: 11153282.6
(22) Anmeldetag: 04.02.2011
(51) Int. Cl.: A61B 17/08, A61B 17/064, A61B 17/00

(54) **Medizinische Fixiereinrichtung**
Medical fixing device
Dispositif de fixation médical

(30) Priorität: 05.02.2010 DE 102010000320
(43) Veröffentlichungstag der Anmeldung: 10.08.2011
(73) Patentinhaber: AESCULAP AG, 78532 Tuttlingen (DE)
(72) Erfinder: Wimber, Johannes, 78187 Geisingen (DE)
(74) Vertreter: Hoeger, Stellrecht & Partner Patentanwälte mbB

(56) Entgegenhaltungen:
- WO-A2-03/071962
- WO-A2-2007/089843
- US-A- 5 026 390
- US-A- 5 531 760
- US-A- 5 618 311
- US-A1- 2009 318 956

## Beschreibung

Die Erfindung betrifft eine medizinische Fixiereinrichtung mit den Merkmalen des Oberbegriffs von Anspruch 1.
Zur Fixierung zweier Körpergewebe aneinander, die voneinander durch einen Spalt getrennt sind, ist es beispielsweise bekannt, diese miteinander zu verkleben oder zu vernähen. Dies hat sich in der Praxis bewährt. Dennoch wäre es wünschenswert, die Körpergewebe auf benutzerfreundlichere Weise miteinander verbinden zu können. Dies gilt insbesondere für schlecht zugängliche Nahtstellen im Körperinnern.
Die US 5,531,760 beschreibt eine gattungsgemäße medizinische Fixiereinrichtung entsprechend dem Oberbegriff des Anspruchs 1. Die Fixiereinrichtung umfasst ein plattenförmiges Halteelement, von dem zweimal zwei im Abstand zueinander angeordnete Fixierelemente abstehen. Die freien Enden der Fixierelemente bilden Fixierglieder zum Eindringen in miteinander zu verbindende Körpergewebe. Um das Einbringen der Fixierglieder in miteinander zu verbindende Körpergewebe zu erleichtern, ist das Halteelement verformbar, so dass die Fixierelemente relativ zueinander eine gespreizte Einbringstellung einnehmen können. Durch das elastisch verformbare Halteelement sind die Fixierelemente relativ zueinander vorgespannt, so dass das Halteelement ein Vorspannelement ausbildet, durch dessen Rückverformung die Fixierelemente in eine Fixierstellung überführt werden können, die sie im die Körpergewebe miteinander verbindenden Zustand einnehmen.
Eine in funktioneller Hinsicht sehr ähnliche medizinische Fixiereinrichtung ist in der US 5,026,390 beschrieben. Ein Halteelement der Fixiereinrichtung ist bügelförmig ausgestaltet und elastisch verformbar. Von einander gegenüberliegenden Enden des Halteelementes stehen Fixierelemente mit Fixiergliedern ab. Durch Verformen des Bügels können die Fixierelemente relativ zueinander in eine Einbringstellung gespreizt werden. Unter der Vorspannung des Bügels werden die Fixierelemente einander wieder angenähert, um zwei Körpergewebe miteinander zu verbinden.

Die WO 03/071962 A2 beschreibt eine medizinische Fixiereinrichtung mit zwei in sich elastisch verformbaren Fixierelementen, deren jeweils einander gegenüberliegende Enden Fixierglieder ausbilden. Mittig sind die Fixierelemente miteinander durch ein Halteelement in Gestalt eines starren Rings verbunden. In funktioneller Hinsicht gleichartig ausgestaltet ist eine in der US 2009/0318956 A1 beschriebene Fixiereinrichtung. Die Fixiereinrichtung weist zwei nadelförmige Fixierelemente auf, deren Enden jeweils verformbare Fixierglieder ausbilden. Die Fixierelemente sind mittig mittels eines starren Halteelementes in Gestalt eines Ringes miteinander verbunden.

In der US 5,618,311 ist eine einstückige medizinische Fixiereinrichtung beschrieben in Gestalt eines durchbrochenen Ringes. Die der Durchbrechung zugewandten Enden des Ringes bilden nadelförmige Fixierglieder zum Eindringen in miteinander zu verbindende Körpergewebe.

Eine weitere medizinische Fixiereinrichtung ist in der WO 2007/089843 A2 beschrieben. Sie weist zwei relativ zueinander nadelförmige, spreizbare Fixierelemente auf, an denen eine Öse festgelegt ist.

Aufgabe der vorliegenden Erfindung ist es, eine einfach handhabbare medizinische Fixiereinrichtung bereitzustellen, mit der sich zwei Körpergewebe auf benutzerfreundlichere Weise aneinander fixieren lassen.

Diese Aufgabe wird bei einer gattungsgemäßen medizinischen Fixiereinrichtung erfindungsgemäß mit den Merkmalen des kennzeichnenden Teils von Anspruch 1 gelöst.

Die erfindungsgemäße Fixiereinrichtung umfasst zwei relativ zueinander bewegliche Fixierelemente, die mittels eines Halteelementes zusammengehalten sind. In einer Einbringstellung kann die Fixiereinrichtung, beispielsweise mittels einer geeigneten Handhabungseinrichtung, in einen zwischen den miteinander zu verbindenden Körpergeweben gebildeten Spalt eingebracht werden. Das erste Fixierelement kann mittels des mindestens einen ersten Fixiergliedes in ein Körpergewebe eindringen und das zweite Fixierelement mittels des mindestens einen zweiten Fixierelementes in das andere Körpergewebe. Dies gibt die Möglichkeit, die Fixiereinrichtung an den beiden Körpergeweben zu verankern. Das Halteelement dient nicht nur zum Zusammenhalten der beiden Fixierelemente, sondern es bildet auch ein Vorspannelement aus. Unter der Wirkung dieses Vorspannelementes sind die beiden Fixierelemente relativ zueinander in der Einbringstellung bezüglich ihrer Fixierstellung vorgespannt, d.h. die Wirkung des Vorspannelementes ist derart, dass es, sofern die Fixierelemente nicht in der Einbringstellung gehalten werden, eine Überführung der Fixierelemente in die Fixierstellung bewirkt. In der Fixierstellung der Fixierelemente weisen das mindestens eine erste und das mindestens eine zweite Fixierglied einen geringeren Abstand voneinander auf als in der Einbringstellung. Dies führt dazu, dass die beiden miteinander zu verbindenden Körpergewebe in Folge der Wirkung des Vorspannelementes einander angenähert und dadurch zuverlässig aneinander fixiert werden. Somit kann mittels der erfindungsgemäßen Fixiereinrichtung gewissermaßen "automatisch" eine Fixierung der Körpergewebe aneinander erfolgen, wenn ein Operateur die Fixierelemente nicht mehr in ihrer Einbringstellung hält. Dadurch erweist sich die Fixiereinrichtung als einfach handhabbar. Zudem weist die Fixiereinrichtung eine einfache konstruktive Ausgestaltung auf, weil das Halteelement mit dem Zusammenhalten der Fixierelemente und dem Vorspannen der Fixierelemente relativ zueinander zwei Funktionen ausübt.

Insbesondere sind aufgrund der Wirkung des Vorspannelementes in der Einbringstellung des ersten und des zweiten Fixierelementes das mindestens eine erste Fixierglied und das mindestens eine zweite Fixierglied relativ zueinander vorgespannt.

Als vorteilhaft hat es sich erwiesen, dass das erste Fixierelement und das zweite Fixierelement einander an einem Überkreuzungsbereich der Fixiereinrichtung überkreuzen und dass das mindestens eine erste Fixierglied und das mindestens eine zweite Fixierglied jeweils einen Abstand zum Überkreuzungsbereich aufweisen. Dies gibt die Möglichkeit, dass das erste Fixierelement und das zweite Fixierelement relativ zueinander unterschiedliche Spreizstellungen einnehmen können. In einer Spreizstellung können sie die Einbringstellung einnehmen und in einer weiteren Spreizstellung die Fixierstellung. Dadurch, dass das mindestens eine erste und das mindestens eine zweite Fixierglied zum Überkreuzungsbereich einen Abstand aufweisen, wird bei gegeneinander spreizbaren Fixierelementen sichergestellt, dass der Abstand des mindestens einen ersten und des mindestens einen zweiten Fixiergliedes voneinander in der Fixierstellung geringer ist als in der Einbringstellung. Dies ermöglicht eine einfache konstruktive Ausgestaltung der Fixiereinrichtung.

Es ist günstig, dass das erste Fixierelement und das zweite Fixierelement relativ zueinander verschwenkbar ausgebildet sind. Dadurch kann unter Erzielung einer einfachen konstruktiven Ausgestaltung der Fixiereinrichtung deren zuverlässige Funktion sichergestellt werden. Durch Verschwenken relativ zueinander können die Fixierelemente von der Einbringstellung in die Fixierstellung überführt werden. Die Schwenkachse, um die die Fixierelemente relativ zueinander verschwenkbar sind, ist günstigerweise parallel ausgerichtet zu jeweiligen, dem jeweils anderen Körpergewebe zugewandten Oberflächen der miteinander zu verbindenden Körpergewebe. Die Schwenkachse verläuft durch den vorstehend erwähnten Überkreuzungsbereich des ersten Fixierelementes und des zweiten Fixierelementes.

Günstig ist es, wenn das erste Fixierelement und/oder das zweite Fixierelement längserstreckt ausgebildet ist. Dadurch kann der Fixiereinrichtung eine einfache Konstruktion verliehen werden.

Als vorteilhaft hat es sich erwiesen, wenn das mindestens eine erste Fixierglied endseitig am ersten Fixierelement angeordnet ist und/oder wenn das mindestens eine zweite Fixierglied endseitig am zweiten Fixierelement angeordnet ist. Dies ermöglicht zum einen eine einfache konstruktive Ausgestaltung der Fixiereinrichtung, und zum anderen erleichtert es das Eindringen des mindestens einen ersten und/oder des mindestens einen zweiten Fixiergliedes in je eines der Körpergewebe.

Von Vorteil ist es, wenn das mindestens eine erste Fixierglied und/oder das mindestens eine zweite Fixierglied einen sich verjüngenden Abschnitt des ersten Fixierelementes bzw. des zweiten Fixierelementes ausbilden. Bei bestimmungsgemäßem Gebrauch der Fixiereinrichtung kann der sich verjüngende Abschnitt beispielsweise quer zu einem der Körpergewebe ausgerichtet werden, so dass dadurch das Eindringen des mindestens einen ersten und/oder des mindestens einen zweiten Fixiergliedes in je eines der Körpergewebe erleichtert wird. Insbesondere bilden das mindestens eine erste und/oder das mindestens eine zweite Fixierglied einen spitz zulaufenden Abschnitt. Dieser ist günstigerweise endseitig am ersten Fixierelement bzw. am zweiten Fixierelement angeordnet und bildet eine endseitige Spitze desselben. Dies ermöglicht ein besonders gutes Eindringen des mindestens einen ersten und/oder des mindestens einen zweiten Fixiergliedes in je eines der Körpergewebe. Dadurch lassen sich die Körpergewebe verbessert aneinander fixieren, denn mit dem Überführen der Fixierelemente von der Einbringstellung in die Fixierstellung können die Fixierglieder zusehends tiefer in die Körpergewebe eindringen und darin verankert werden.

Günstig ist es, wenn ein Fixierglied eines Fixierelementes bezüglich eines Fixiergliedes des anderen Fixierelementes eine konvexe Krümmung aufweist. Beispielsweise kann also das mindestens eine erste Fixierglied bezüglich des mindestens einen zweiten Fixiergliedes konvex gekrümmt sein und/oder umgekehrt. Das konvex gekrümmte Fixierglied kann dadurch in der Fixierstellung der Fixierelemente einen Abschnitt desjenigen Körpergewebes, in das es eindringt, welcher dem anderen Körpergewebe zugewandt ist, bogenförmig hintergreifen. Dadurch kann eine zuverlässige Fixierung der Körpergewebe aneinander sichergestellt werden.

Es kann vorgesehen sein, dass ein Fixierglied eines Fixierelementes bezüglich eines Fixiergliedes des anderen Fixierelementes eine konkave Krümmung aufweist. Beispielsweise ist das mindestens eine erste Fixierglied relativ zu dem mindestens einen zweiten Fixierglied konkav gekrümmt und/oder umgekehrt. Es hat sich in der Praxis gezeigt, dass insbesondere in der Einbringstellung der Fixierelemente das konkav gekrümmte Fixierglied auf besonders gute Weise in eines der Körpergewebe eindringen kann. Dies erleichtert das Verankern der Fixiereinrichtung an den Körpergeweben.

Vorzugsweise umfasst das erste Fixierelement zwei Fixierglieder und/oder das zweite Fixierelement umfasst zwei Fixierglieder. Dies ermöglicht eine zuverlässigere Fixierung der beiden Körpergewebe aneinander. Insbesondere kann vorgesehen sein, dass jedes Fixierelement mit jeweils einem Fixierglied in je eines der beiden miteinander zu verbindenden Körpergewebe eindringen kann.

Günstig ist es, wenn die zwei ersten Fixierglieder des ersten Fixierelementes an einander gegenüberliegenden Enden des ersten Fixierelementes angeordnet sind und/oder wenn die zwei zweiten Fixierglieder des zweiten Fixierelementes an einander gegenüberliegenden Enden des zweiten Fixierelementes angeordnet sind. Dies ermöglicht zum einen eine konstruktiv einfache Ausgestaltung der Fixiereinrichtung unter Erzielung einer zuverlässigen Fixierung der beiden Körpergewebe aneinander. Zum anderen ist dadurch auf einfache Weise die Möglichkeit gegeben, dass das erste Fixierelement und/oder das zweite Fixierelement, wie vorstehend beschrieben, mit je einem Fixierglied in je eines der miteinander zu verbindenden Körpergewebe eindringen. Hierfür können die Fixierelemente beispielsweise in der Einbringstellung quer zu den jeweiligen Oberflächen der Körpergewebe ausgerichtet werden.

Ein Fixierelement, das wie vorstehend beschrieben an jedem seiner gegenüberliegenden Enden jeweils ein Fixierglied aufweist, ist günstigerweise nadelförmig ausgestaltet. Die auf diese Weise gebildete Nadel kann an beiden Enden spitz zulaufen, und jedes der spitz zulaufenden Enden der Nadel bildet ein Fixierglied. Dadurch weist die Fixiereinrichtung eine besonders einfache konstruktive Ausgestaltung auf. Die Nadel kann beispielsweise eine S-förmige Gestalt haben, wobei jeweils endseitig an der Nadel angeordnete Fixierglieder eine konvexe oder eine konkave Krümmung aufweisen können.

Vorzugsweise umgreift das Halteelement das erste Fixierelement und das zweite Fixierelement, um das erste Fixierelement und das zweite Fixierelement zusammenzuhalten. Dies erlaubt eine einfache konstruktive Ausgestaltung der Fixiereinrichtung.

Das Halteelement liegt günstigerweise sowohl am ersten Fixierelement als auch am zweiten Fixierelement an. Dadurch kann über das Halteelement eine Kraft auf das erste und das zweite Fixierelement ausgeübt werden.

Als günstig hat es sich erwiesen, wenn das Halteelement das erste Fixierelement und das zweite Fixierelement jeweils mittig zwischen zwei Enden des ersten Fixierelementes und zwei Enden des zweiten Fixierelementes umgreift. Endseitig umfassen das erste und/oder das zweite Fixierelement dann günstigerweise jeweils ein Fixierglied. Insbesondere umgreift das Halteelement das erste und das zweite Fixierelement an dem vorstehend erwähnten Überkreuzungsbereich.

Bislang wurde noch nicht auf die nähere Ausgestaltung des Halteelementes als Vorspannelement eingegangen. Von Vorteil ist es, wenn das Halteelement mindestens ein elastisches Element umfasst oder ausbildet, das in der Einbringstellung des ersten Fixierelementes und des zweiten Fixierelementes einen gespannten Zustand einnimmt und in der Fixierstellung des ersten Fixierelementes und des zweiten Fixierelementes einen entspannteren Zustand einnimmt. Nimmt das elastische Element den gespannten Zustand ein, kann es eine höhere potentielle Energie aufweisen als im entspannteren Zustand. Dies führt zu einer Vorspannung der Fixierelemente relativ zueinander in der Einbringstellung bezüglich ihrer Fixierstellung. Durch Freiwerden der Differenz an potentieller Energie im gespannten Zustand relativ zur potentiellen Energie im entspannteren Zustand können das erste und das zweite Fixierelement mit einer Kraft beaufschlagt werden, um sie von der Einbringstellung in die Fixierstellung zu überführen.

Es kann vorgesehen sein, dass der Operateur dem mindestens einen elastischen Element vor dem Einbringen der Fixiereinrichtung in den Körper hinreichend potentielle Energie zuführt, um es vom entspannteren Zustand in den gespannten Zustand zu überführen. Dies kann beispielsweise dadurch erfolgen, dass der Operateur die Fixierelemente mittels einer Handhabungseinrichtung von der Fixierstellung in die Einbringstellung unter Spannung oder Dehnung des mindestens einen elastischen Elementes überführt und in dieser beim Einbringen in den Körper hält.

Das mindestens eine elastische Element ist günstigerweise in sich geschlossen ausgebildet. Dadurch kann es die Fixierelemente auf konstruktiv einfache Weise umgreifen und speziell umgeben.

Bei einer einfachen konstruktiven Ausgestaltung der Fixiereinrichtung ist das Halteelement als ein das erste Fixierelement und das zweite Fixierelement umgebender O-Ring ausgestaltet. Zum einen ist der O-Ring in sich geschlossen, so dass er die Fixierelemente umgeben und sie dadurch zusammenhalten kann. Zum anderen bildet der O-Ring ein elastisches Element. In der Einbringstellung der Fixierelemente kann er einen gespannten Zustand einnehmen. Beispielsweise wird er dabei durch die Fixierelemente unter Vergrößerung seiner Querschnittsfläche aufgeweitet. Aufgrund seiner elastischen Eigenschaft kann sich der O-Ring wieder unter Freigabe der gespeicherten potentiellen Energie und unter Verringerung seiner Querschnittsfläche zusammenziehen. Weil er die Fixierelemente umgibt, werden diese dadurch mit einer Kraft beaufschlagt und können von der Einbringstellung in die Fixierstellung überführt werden, in welcher der O-Ring einen entspannteren Zustand einnehmen kann. Insbesondere umgibt der O-Ring die Fixierelemente formschlüssig.

Bei einer vorteilhaften konstruktiven Ausgestaltung der Fixiereinrichtung umgibt der O-Ring die Fixierelemente an deren vorstehend beschriebenem Überkreuzungsbereich.

Der O-Ring kann einen kreisförmigen Querschnitt aufweisen, es ist jedoch auch denkbar, dass er einen andersartigen Querschnitt aufweist.

Von Vorteil ist es, wenn das erste Fixierelement ein erstes Anlageglied für das zweite Fixierelement aufweist und wenn das zweite Fixierelement ein zweites Anlageglied für das erste Fixierelement aufweist und wenn das erste Fixierelement und das zweite Fixierelement über das erste Anlageglied und das zweite Anlageglied aneinander anliegen. Dadurch kann eine zuverlässige Positionierung der Fixierelemente relativ zueinander und damit eine zuverlässige Funktion der Fixiereinrichtung sichergestellt werden. Die zwei Fixierelemente können sich durch die Anlageglieder beim Überführen der Fixierelemente von der Einbringstellung in die Fixierstellung gegenseitig führen.

Es kann vorgesehen sein, dass das erste Anlageglied mittig zwischen zwei Enden des ersten Fixierelementes an diesem angeordnet ist und/oder dass das zweite Anlageglied mittig zwischen zwei Enden des zweiten Fixierelementes an diesem angeordnet ist. Hierbei sind an den jeweiligen Enden des ersten Fixierelementes und/oder des zweiten Fixierelementes vorzugsweise jeweils erste bzw. zweite Fixierglieder gebildet.

Insbesondere kann vorgesehen sein, dass die Position des ersten Anlagegliedes und/oder des zweiten Anlagegliedes den vorstehend erwähnten Überkreuzungsbereich des ersten Fixierelementes und des zweiten Fixierelementes definiert, d.h. die Fixierelemente können einander in dem Bereich überkreuzen, in dem die beiden Anlageglieder angeordnet sind.

Von Vorteil ist es, wenn das erste Anlageglied und/oder das zweite Anlageglied planar ausgestaltet ist. Dadurch können das erste Fixierelement und das zweite Fixierelement flächig aneinander anliegen. Dies sichert eine zuverlässige Funktion der Fixiereinrichtung, speziell wenn die Fixierelemente relativ zueinander verschwenkbar ausgebildet sind.

Bevorzugt umfasst das erste Fixierelement ein erstes Lagerelement, das mit einem vom zweiten Fixierelement umfassten zweiten Lagerelement zur Lagerung des ersten Fixierelementes und des zweiten Fixierelementes aneinander zusammenwirkt. Dies ermöglicht eine zuverlässigere Funktion der Fixiereinrichtung, denn beim Überführen der Fixierelemente von der Fixierstellung in die Einbringstellung und/oder umgekehrt können die Fixierelemente aneinander gelagert sein. Insbesondere sind die Fixierelemente mittels der Lagerelemente schwenkbar aneinander gelagert, wobei die Lagerelemente eine Schwenkachse definieren können. Beispielsweise ist hierfür ein Lagerelement als Lagerzapfen ausgebildet und das andere Lagerelement als Ausnehmung, in die der Lagerzapfen insbesondere formschlüssig eingreifen kann. Möglich wäre es auch, dass ein Lagerelement als konvexer Vorsprung ausgebildet ist, der in das andere Lagerelement in Form einer konvexen Prägung eingreift.

Auch die vorstehend erwähnten Anlageglieder können Lagerelemente zur Lagerung der Fixierelemente aneinander ausbilden.

Als günstig hat es sich erwiesen, wenn ein Fixierelement eine in dem anderen Fixierelement gebildete Durchbrechung durchgreift und insbesondere formschlüssig durchgreift. Quer zur Erstreckung der Durchbrechung kann auf diese Weise eine zuverlässige Positionierung der Fixierelemente relativ zueinander sichergestellt werden. Insbesondere können die Fixierelemente gemeinsam einen Kastenschluss ausbilden. Die die Durchbrechung einfassenden Wandungen des einen Fixierelementes können Anlageglieder und Lagerelemente für das die Durchbrechung durchgreifende Fixierelement ausbilden.

Vorzugsweise weisen das erste Fixierelement und das zweite Fixierelement jeweils mindestens ein erstes Anschlagsglied für das jeweils andere Fixierelement auf zur Begrenzung einer Bewegung des ersten Fixierelementes und des zweiten Fixierelementes relativ zueinander beim Überführen des ersten Fixierelementes und des zweiten Fixierelementes von der Einbringstellung in die Fixierstellung. Dadurch kann sichergestellt werden, dass die Fixierelemente in der Fixierstellung relativ zueinander eine definierte Position einnehmen. In dieser Position wirken die jeweiligen ersten Anschlagsglieder des ersten und des zweiten Fixierelementes zusammen, um den Bewegungsbereich der Fixierelemente relativ zueinander zu beschränken. Dies sichert eine zuverlässige Funktion der Fixiereinrichtung.

In entsprechender Weise ist es von Vorteil, wenn das erste Fixierelement und das zweite Fixierelement jeweils mindestens ein zweites Anschlagsglied für das jeweils andere Fixierelement aufweisen zur Begrenzung einer Bewegung des ersten Fixierelementes und des zweiten Fixierelementes relativ zueinander beim Überführen des ersten Fixierelementes und des zweiten Fixierelementes von der Fixierstellung in die Einbringstellung. Dadurch kann sichergestellt werden, dass - beispielsweise vor dem Einbringen der Fixiereinrichtung in den Körper - die Fixierelemente relativ zueinander eine definierte Position einnehmen können. In dieser Position wirken die jeweiligen zweiten Anschlagsglieder zusammen, um den Bewegungsbereich der Fixierelemente relativ zueinander zu begrenzen. Auch dies sichert eine zuverlässige Funktion der Fixiereinrichtung.

Zur Erzielung einer einfachen Konstruktion der Fixiereinrichtung ist es günstig, wenn das erste Fixierelement und/oder das zweite Fixierelement und/oder das Halteelement einstückig ausgebildet ist.

Vorzugsweise sind das erste Fixierelement und das zweite Fixierelement identisch ausgebildet. Dies verringert die Herstellungskosten für die Fixiereinrichtung und verleiht ihr eine einfachere Konstruktion.

Vorzugsweise ist mindestens ein Fixierelement in sich symmetrisch ausgebildet, und insbesondere können beide Fixierelemente in sich symmetrisch ausgebildet sein. Dies verringert die Herstellungskosten für die Fixiereinrichtung und es vereinfacht ihre konstruktive Ausgestaltung.

Es kann vorgesehen sein, dass die Fixiereinrichtung teilweise und insbesondere vollständig aus einem resorbierbaren Material besteht.

Die nachfolgende Beschreibung bevorzugter Ausführungsformen der Erfindung dient im Zusammenhang mit der Zeichnung der näheren Erläuterung der Erfindung. Es zeigen:
- Figur 1A:: eine perspektivische Darstellung einer ersten bevorzugten Ausführungsform einer erfindungsgemäßen Fixiereinrichtung mit zwei Fixierelementen, die relativ zueinander eine Fixierstellung einnehmen;
- Figur 1B:: eine Explosionsdarstellung der Fixiereinrichtung aus Figur 1;
- Figur 2:: die Fixiereinrichtung aus Figur 1, bei der die Fixierelemente relativ zueinander eine Einbringstellung einnehmen;
- Figur 3:: die Fixiereinrichtung aus Figur 1, bei der die Fixierelemente relativ zueinander mittels einer teilweise dargestellten Handhabungseinrichtung in einer Einbringstellung gehalten werden;
- Figur 4:: die Fixiereinrichtung aus Figur 1, deren Fixierelemente eine Einbringstellung einnehmen, beim Einbringen zwischen zwei miteinander zu verbindende Körpergewebe, teilweise geschnitten;
- Figur 5:: eine Abbildung entsprechend Figur 4, wobei die Fixierelemente von der Einbringstellung in eine Fixierstellung überführt werden;
- Figur 6:: eine Darstellung entsprechend Figur 4, wobei die Fixierelemente eine Fixierstellung einnehmen und die beiden Körpergewebe aneinander fixiert sind;
- Figur 7:: eine Darstellung entsprechend Figur 6 mit einer zweiten bevorzugten Ausführungsform einer erfindungsgemäßen Fixiereinrichtung;
- Figur 8:: eine Darstellung entsprechend Figur 1B mit einer dritten bevorzugten Ausführungsform einer erfindungsgemäßen Fixiereinrichtung und
- Figur 9:: eine perspektivische Darstellung einer vierten bevorzugten Ausführungsform einer erfindungsgemäßen Fixiereinrichtung, teilweise in Explosionsdarstellung mit zwei relativ zueinander eine Fixierstellung einnehmenden Fixierelementen.

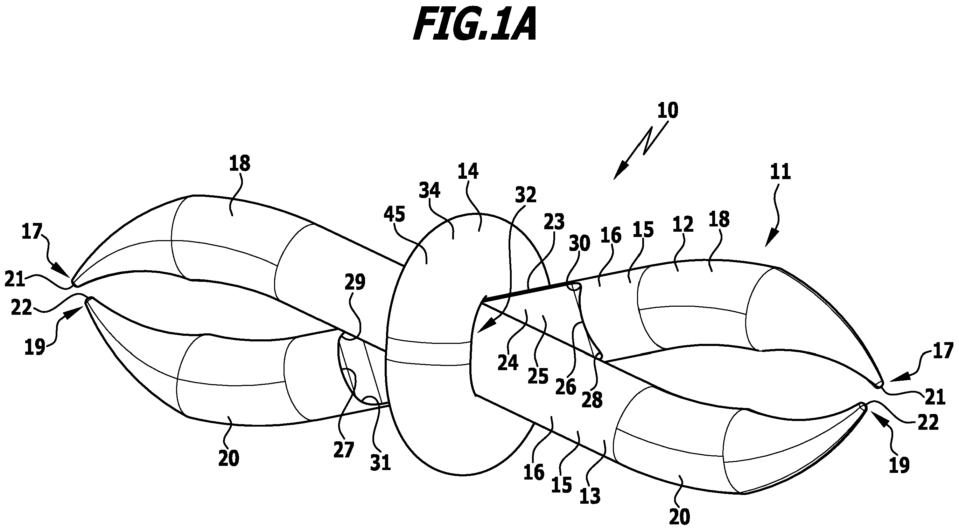

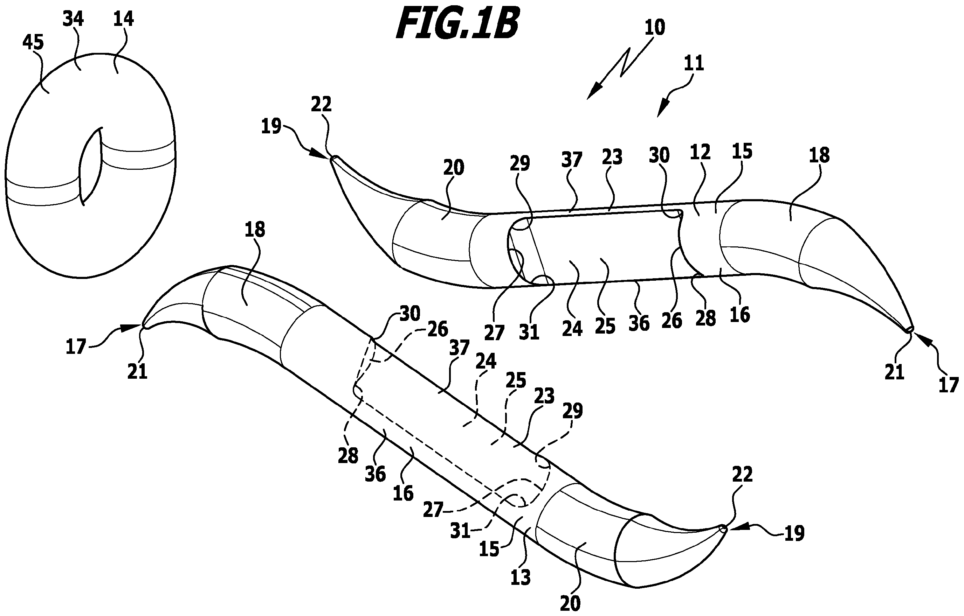

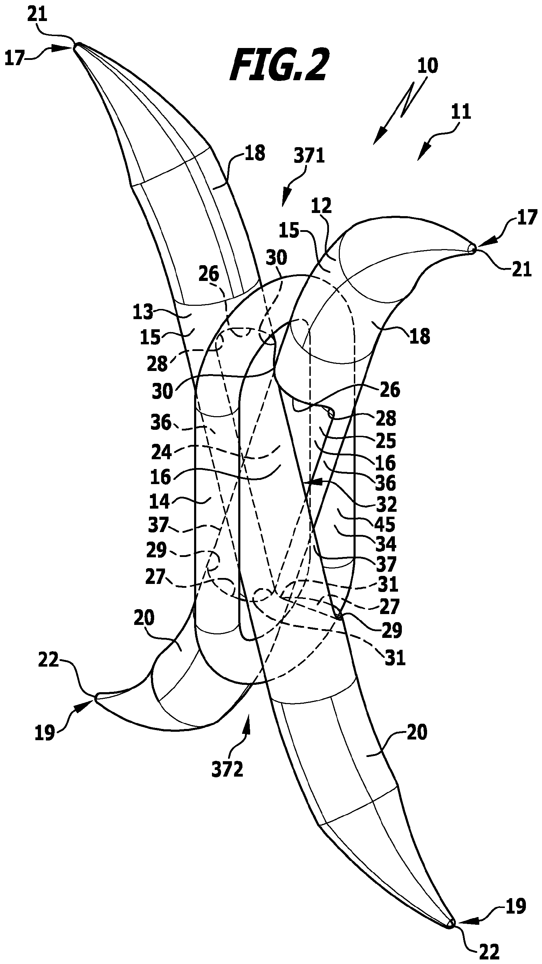

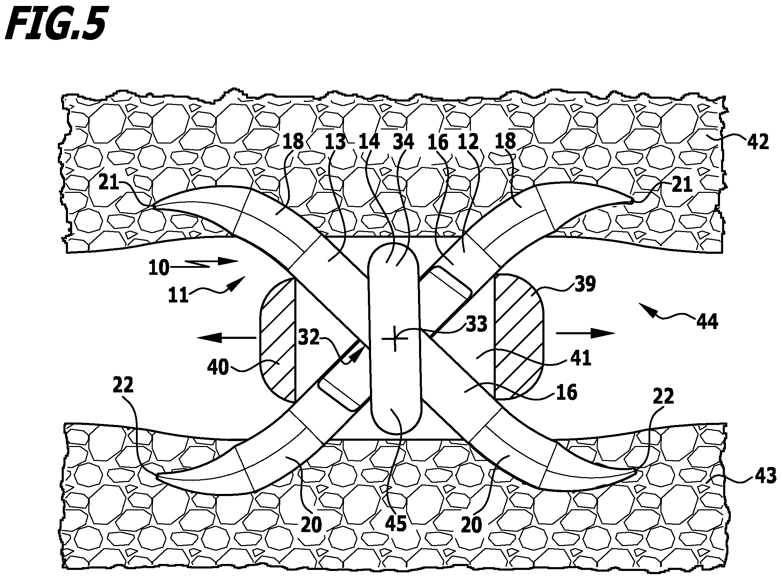

Eine erste bevorzugte Ausführungsform einer erfindungsgemäßen Fixiereinrichtung ist in Figur 1A perspektivisch dargestellt und in Figur 1B in einer perspektivischen Explosionsdarstellung entsprechend der Figur 1A dargestellt. Die Fixiereinrichtung ist darin sowie in den Figuren 2 bis 6 jeweils mit dem Bezugszeichen 10 belegt. Die Fixiereinrichtung 10, nachfolgend als Klammer 11 bezeichnet, umfasst drei jeweils einstückig ausgebildete Bauteile, nämlich ein erstes Fixierelement 12, ein zweites Fixierelement 13 sowie ein Halteelement 14.

Die Fixierelemente 12 und 13 sind identisch zueinander ausgebildet, weswegen nachfolgend nur auf die einzelnen Merkmale des ersten Fixierelementes 12 eingegangen wird. Diese sind bei dem zweiten Fixierelement 13 mit identischen Bezugszeichen versehen. Zusätzlich ist jedes der Fixierelemente 12, 13 in sich symmetrisch ausgebildet.

Das Fixierelement 12 ist ausgestaltet in Form einer S-förmigen Nadel 15 mit einem mittleren Abschnitt 16, an den sich in Richtung eines ersten Endes 17 der Nadel 15 ein Fixierglied 18 anschließt. In entsprechender Weise schließt sich an den mittleren Abschnitt 16 in Richtung eines zweiten Endes 19 der Nadel 15 ein Fixierglied 20 an. Ausgehend vom mittleren Abschnitt 16 verjüngt sich das Fixierelement 12 jeweils an den Fixiergliedern 18 und 20, d.h. diese bilden jeweils sich verjüngende Abschnitte des Fixierelementes 12. Die Verjüngung der Fixierglieder 18 und 20 erfolgt dabei derart, dass sie in Richtung der Enden 17 bzw. 19 spitz zulaufen und an den Enden 17 bzw. 19 Spitzen 21 bzw. 22 des Fixierelementes 12 bilden. Relativ zum mittleren Abschnitt 16, der eine geradlinige Erstreckung aufweist, sind die Fixierglieder 18 und 20 jeweils gekrümmt ausgebildet, und zwar ausgehend vom mittleren Abschnitt 16 in einander entgegengesetzten Richtungen. Dadurch wird die S-förmige Gestalt der längserstreckten Nadel 15 hervorgerufen.

Die Nadel 15 weist einen runden Querschnitt auf, ausgenommen an einem Bereich 23 des mittleren Abschnittes 16, an welchem Bereich 23 eine Materialausnehmung 24 gebildet ist. Der Bereich 23 befindet sich mittig zwischen den Enden 17 und 19, und die Materialausnehmung 24 erstreckt sich ungefähr über ein Drittel des Durchmessers der Nadel 15 im mittleren Abschnitt 16.

Im Bereich der Materialausnehmung 24 bildet der mittlere Abschnitt 16 ein Anlageglied 25, das planar ausgestaltet ist. In Richtung der Fixierglieder 18 und 20 wird die Materialausnehmung 24 durch Ränder 26 bzw. 27 begrenzt. An den Rändern 26 und 27 umfasst der mittlere Abschnitt 16 zwei Paare von Anschlagsgliedern, und zwar ein erstes Paar von Anschlagsgliedern 28 und 29 sowie ein zweites Paar von Anschlagsgliedern 30 sowie 31. Die Anschlagsglieder 28 und 29 sind an den den Spitzen 21 bzw. 22 zugewandten Seiten der Ränder 26 bzw. 27 angeordnet. Die Anschlagsglieder 30 und 31 sind an den den Spitzen 21 bzw. 22 abgewandten Seiten der Ränder 26 bzw. 27 angeordnet.

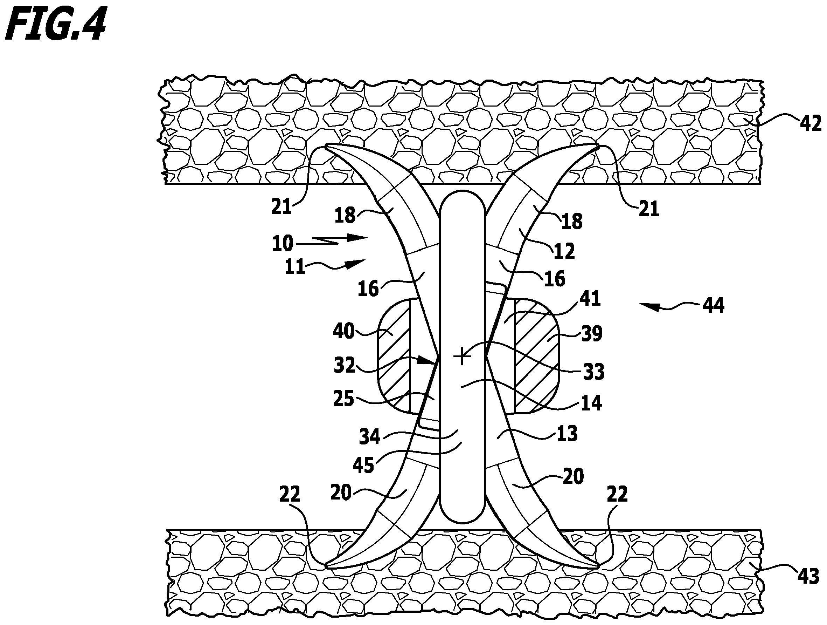

Zur Ausbildung der Klammer 11 sind die Fixierelemente 12 und 13 mittels ihrer jeweiligen Anlageglieder 25 aneinander angelegt. Weil die Fixierelemente 12 und 13 identisch ausgestaltet sind, führt dies dazu, dass sie sich an ihren jeweiligen Bereichen 23 überkreuzen, so dass die jeweiligen Bereiche 23 einen Überkreuzungsbereich 32 der Fixierelemente 12 und 13 definieren. An den Anlagegliedern 25 können die Fixierelemente 12 und 13 jeweils aneinander entlang gleiten und sich gegenseitig führen. Insbesondere können sie relativ zueinander verschwenkt werden um eine Schwenkachse 33 (Figuren 4 bis 6). Die Schwenkachse 33 ist jeweils senkrecht zu der von den jeweiligen Anlagegliedern 25 definierten Ebenen der Fixierelemente 12 und 13 ausgerichtet. Sie weist von den jeweiligen Enden 17 und 19 der Fixierelemente 12 und 13 jeweils identische Abstände auf.

Zur Ausbildung der Klammer 11 werden die Fixierelemente 12 und 13 am Überkreuzungsbereich 32 mittels des Halteelementes 14 zusammengehalten. Das Halteelement 14 umgreift die Fixierelemente 12 und 13, insbesondere umgibt das Halteelement 14 die Fixierelemente 12 und 13 am Überkreuzungsbereich 32 formschlüssig.

Das Halteelement 14 ist ausgestaltet in Form eines O-Ringes 34, der aus einem elastischen Material gefertigt ist. Er kann einen entspannten Zustand einnehmen (Figur 1B), in dem die in ihm gespeicherte potentielle Energie minimiert ist, d.h. der O-Ring 34 ist so weit wie möglich zusammengezogen und weist eine minimale Querschnittsfläche auf. Der O-Ring 34 ist vorliegend in seinem entspannten Zustand von kreisförmigem Querschnitt. Er könnte allerdings auch einen andersartigen Querschnitt aufweisen.

Wenn der O-Ring 34 indessen die Fixierelemente 12 und 13 umgibt, wird er durch deren mittlere Abschnitte 16 ein wenig aufgeweitet. Durch seine elastische Eigenschaft kann er die Fixierelemente 12 und 13 zusammenhalten (Figur 1A).

Ist dies der Fall, beaufschlagt der O-Ring 34 die Fixierelemente 12 und 13 relativ zueinander mit einer Kraft, die dazu führt, dass die Fixierelemente 12 und 13 über ihre jeweiligen Anschlagsglieder 28 und 29 aneinander anliegen. Dabei liegt das Anschlagsglied 28 des Fixierelementes 12 am Anschlagsglied 29 des Fixierelementes 13 an und umgekehrt, und die Spitzen 21 und 22 des Fixierelementes 12 berühren beinahe die Spitzen 22 bzw. 21 des Fixierelementes 13. Die Anschlagsglieder 28 und 29 stellen sicher, dass die Fixierelemente 12 und 13 relativ zueinander in dieser klar definierten Stellung verbleiben und nicht unter der Wirkung des O-Ringes 34 weiter relativ zueinander bewegt werden.

Ausgehend von dieser Stellung der Fixierelemente 12 und 13, welche eine Fixierstellung definiert, kann die Klammer 11 in eine Einbringstellung überführt werden (Figur 2). Dies erfolgt durch Verschwenken der Fixierelemente 12 und 13 relativ zueinander um die Schwenkachse 33. Dadurch geraten die Fixierglieder 18 und 20 des Fixierelementes 12 zu den Fixiergliedern 20 bzw. 18 des Fixierelementes 13 in einen Abstand, der größer ist als ihr jeweiliger Abstand in der Fixierstellung der Klammer 11.

Zur Überführung der Fixierelemente 12 und 13 von der Fixierstellung in die Einbringstellung können die Fixierelemente 12 und 13 beispielsweise nahe den Anschlagsgliedern 28 an jeweiligen Seitenbereichen 36 des mittleren Abschnittes 16 mit aufeinander zugewandten Kräften beaufschlagt werden. Ergänzend oder alternativ können die Fixierelemente 12 und 13 beispielsweise nahe den Anschlagsgliedern 29 an jeweiligen Seitenbereichen 37 der mittleren Abschnitte 16 mit aufeinander zugewandten Kräften beaufschlagt werden.

In der Einbringstellung der Fixierelemente sind spitzwinklige Zwickel 371 und 372 zwischen den Fixierelementen 12 und 13 gebildet. Der Zwickel 371 wird durch den Seitenbereich 37 und das Fixierglied 18 des Fixierelementes 12 sowie durch den Seitenbereich 36 und das Fixierglied 18 des Fixierelementes 13 begrenzt. Der Zwickel 372 wird durch den Seitenbereich 36 und das Fixierglied 20 des Fixierelementes 12 und den Seitenbereich 37 und das Fixierglied 20 des Fixierelementes 13 begrenzt.

Beim Verschwenken der Fixierelemente 12 und 13 relativ zueinander um die Schwenkachse 33 wird der O-Ring 34 aufgeweitet. Dies liegt daran, dass der O-Ring 34 jeweils an den Seitenbereichen 36 und 37 der Fixierelemente 12 bzw. 13 entlang gleitet. Er durchgreift die Zwickel 371 und 372. Das Aufweiten des O-Ringes 34 erfolgt entgegen seiner elastischen Rückstelleigenschaft, d.h. dem O-Ring 34 wird zum Aufweiten potentielle Energie zugeführt. Diese bleibt so lange im O-Ring 34 gespeichert, wie sich die Fixierelemente 12 und 13 relativ zueinander in der Einbringstellung befinden. In der Einbringstellung der Fixierelemente 12 und 13 werden diese vom O-Ring 34 weiterhin nahezu formschlüssig umgeben und zusammengehalten.

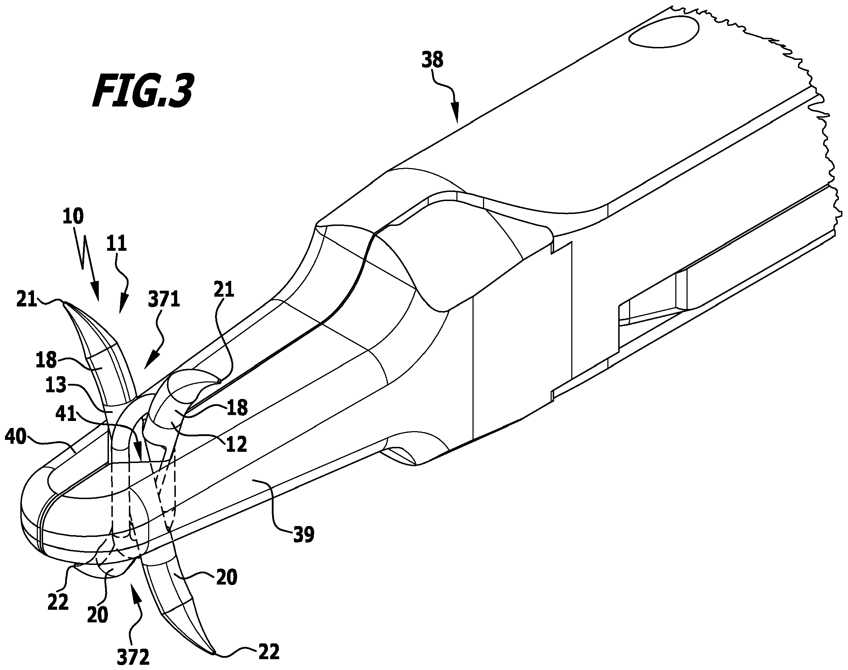

Um die Fixierelemente 12 und 13 in die Einbringstellung zu überführen und diese darin zu halten, kann eine Handhabungseinrichtung 38 vorgesehen sein mit zwei relativ zueinander beweglich ausgebildeten Maulteilen 39 und 40 (Figuren 3 bis 5). Eine derartige Handhabungseinrichtung 38 ist an sich bekannt. Deswegen wird sie vorliegend nur dahingehend erläutert, dass zwischen den Maulteilen 39 und 40 eine Aufnahme 41 für die Klammer 11 gebildet sein kann. In die Aufnahme 41 kann die Klammer 11 beispielsweise in einem gespreizten Zustand der Maulteile 39 und 40 relativ zueinander eingeführt werden. Werden die Maulteile 39 und 40 einander angenähert, führt dies dazu, dass die Seitenbereiche 36 und 37 der Fixierelemente 12 und 13 jeweils mit aufeinander zugewandten Kräften beaufschlagt werden. Dies führt zum Überführen der Fixierelemente 12 und 13 von der Fixierstellung in die Einbringstellung unter Aufweitung des O-Ringes 34.

In der Einbringstellung können die Fixierelemente 12 und 13 mittels ihrer jeweiligen Anschlagsglieder 30 und 31 aneinander anliegen, d.h. es liegen jeweils die Anschlagsglieder 30 sowie die Anschlagsglieder 31 der Fixierelemente 12 und 13 aneinander an. Dadurch ist sichergestellt, dass die Fixierelemente 12 und 13 nicht so weit gegeneinander unter Aufweitung des O-Ringes 34 gespreizt werden, dass dieser beschädigt wird.

Aufgrund der elastischen Ausgestaltung des O-Ringes 34 sind die Fixierelemente 12 und 13 in ihrer Einbringstellung relativ zueinander bezüglich ihrer Fixierstellung vorgespannt. Aus diesem Grund bildet der O-Ring 34 ein Vorspannelement 45. Insbesondere sind aufgrund der vom O-Ring 34 vermittelten Vorspannung auf die Fixierelemente 12 und 13 auch die jeweiligen Fixierglieder 18 und 20 relativ zueinander vorgespannt. Und zwar ist das Fixierglied 18 des Fixierelementes 12 relativ zum Fixierglied 20 des Fixierelementes 13 vorgespannt, und das Fixierglied 20 des Fixierelementes 12 ist relativ zum Fixierglied 18 des Fixierelementes 13 vorgespannt.

Wie insbesondere aus den Figuren 4 bis 6 deutlich wird, kann die Klammer 11 auf benutzerfreundliche Weise zur Fixierung von zwei Körpergeweben 42 und 43 aneinander eingesetzt werden. Bei den Körpergeweben 42 und 43 kann es sich beispielsweise um Menisken im Kniegelenk handeln.

Zunächst kann die Klammer 11, die zwischen den Maulteilen 39 und 40 in der Aufnahme 41 gehalten ist, in einen Zwischenraum 44 zwischen den Körpergeweben 42 und 43 eingebracht werden. Diese werden hierfür relativ zueinander gespreizt. Aufgrund der spitz ausgestalteten Fixierglieder 18 und 20 können die Fixierelemente 12 und 13 jeweils partiell in die Körpergewebe 42 und 43 eindringen. Insbesondere dringt das Fixierelement 12 mit den Fixiergliedern 18 und 20 in die Körpergewebe 42 bzw. 43. ein. In entsprechender Weise dringt das Fixierelement 13 mit den Fixiergliedern 18 und 20 in die Körpergewebe 42 bzw. 43 ein (Figur 4).

Werden nachfolgend die Maulteile 39 und 40 unter Erweiterung der Aufnahme 41 in bekannter Weise relativ zueinander gespreizt, wird die im elastischen O-Ring 34 gespeicherte potentielle Energie frei. Der O-Ring 34 zieht sich ausgehend von seinem gespannten Zustand in der Einbringstellung der Fixierelemente 12 und 13 in einen entspannteren Zustand zusammen. Die dabei frei werdende potentielle Energie wird, da der O-Ring 34 die Fixierelemente 12 und 13 umgibt, auf diese übertragen. Dies führt dazu, dass die Fixierelemente 12 und 13 relativ zueinander um die Schwenkachse 33 verschwenkt werden und von ihrer Einbringstellung in Richtung der Fixierstellung überführt werden. Als Konsequenz nähern sich die Fixierglieder 18 und 20 des Fixierelementes 12 den Fixiergliedern 20 bzw. 18 des Fixierelementes 13 an. Während dieses Vorgangs dringt das erste Fixierelement 12 mit den Fixiergliedern 18 und 20 tiefer in die Körpergewebe 42 bzw. 43 ein. In entsprechender Weise dringt das zweite Fixierelement 13 mit den Fixiergliedern 18 und 20 tiefer in die Körpergewebe 42 bzw. 43 ein.

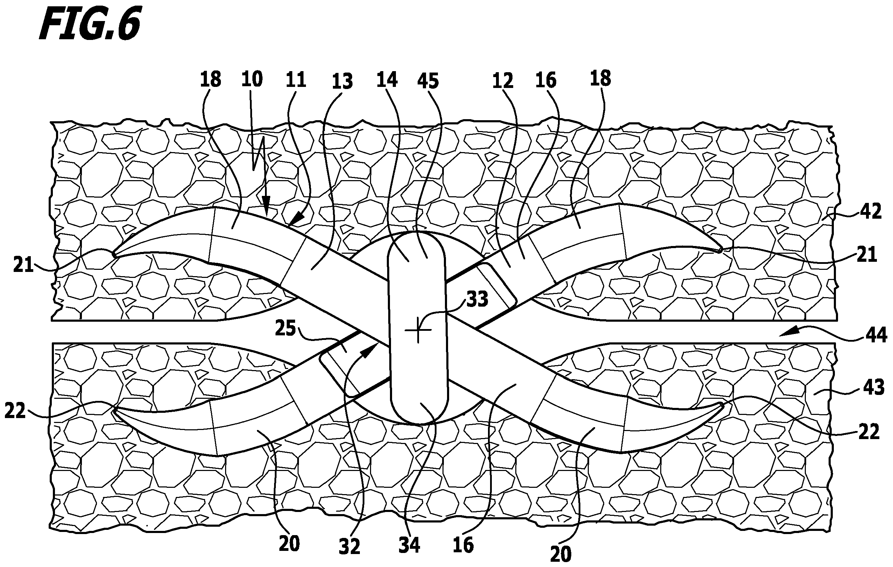

Unter weiterer Wirkung des sich zusammenziehenden O-Ringes 34 setzt sich dieser Eindringvorgang fort. Wird zusätzlich die Handhabungseinrichtung 38 aus dem Zwischenraum 44 entfernt, können dadurch die Körpergewebe 42 und 43 aufeinander zu bewegt werden, weil sich die Fixierglieder 18 und 20 der Fixierelemente 12 und 13 weiter aneinander annähern, während diese unter der Wirkung des sich entspannenden O-Ringes 34 in eine Fixierstellung überführt werden (Figur 6). Dadurch kann eine zuverlässige Fixierung der Körpergewebe 42 und 43 relativ zueinander erzielt werden.

Hierbei geraten, beispielsweise je nach Art der Körpergewebe 42 und 43 und je nach Größe der Klammer 11, nicht notwendigerweise die jeweiligen Anschlagsglieder 28 und 29 der Fixierelemente 12 und 13 aneinander zur Anlage. Der O-Ring 34 wird dabei eine Restspannung aufweisen, so dass die Fixierelemente 12 und 13 zur Sicherung der Fixierung der Körpergewebe 42 und 43 aneinander relativ zueinander weiterhin etwas vorgespannt sind.

Von Vorteil ist es, dass die Fixierglieder 18 und 20 des Fixierelementes 12, bezogen auf die Fixierglieder 20 bzw. 18 des Fixierelementes 13, und umgekehrt, eine konvexe Krümmung aufweisen. Dadurch beschreiben die Fixierglieder 18 und 20 beim Eindringen in die Körpergewebe 42 und 43 jeweils einen Bogen. Infolgedessen werden die Körpergewebe 42 und 43 unter der Wirkung des sich entspannenden O-Rings 34 besonders wirkungsvoll aufeinander zu gezogen. In der Fixierstellung der Fixierelemente 12 und 13 sind die Körpergewebe 42 und 43 aneinander somit besonders zuverlässig fixiert.

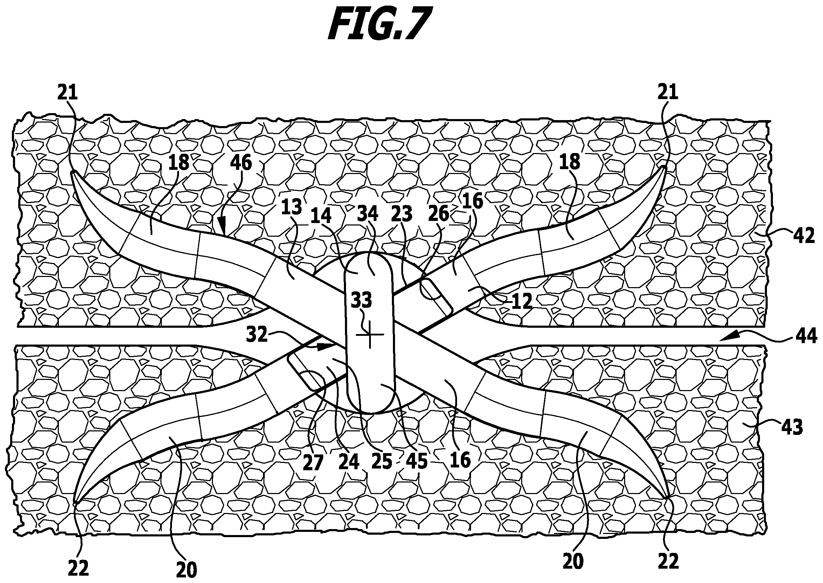

Eine zweite bevorzugte Ausführungsform einer erfindungsgemäßen Fixiereinrichtung ist in Figur 7 in einer der Figur 6 entsprechenden Weise dargestellt und dort insgesamt mit dem Bezugszeichen 46 belegt. Die Fixiereinrichtung 46 ist weitestgehend identisch ausgebildet zur Fixiereinrichtung 10, und für ihre Merkmale werden die identischen Bezugszeichen wie für die Merkmale der Fixiereinrichtung 10 verwendet.

Anders als bei der Fixiereinrichtung 10 sind die Fixierglieder 18 und 20 der Fixierelemente 12 und 13 in Richtung der Enden 17 bzw. 19 in zwei einander entgegengesetzten Richtungen gekrümmt. Dies bedeutet, dass die Fixierglieder 18 und 20 jeweils in sich S-förmig ausgestaltet sind. Dies hat zur Folge, dass die Fixierglieder 18 und 20 des Fixierelementes 12 relativ zu den Fixiergliedern 20 bzw. 18 des Fixierelementes 13 bei der Fixiereinrichtung 46 eine konkave Krümmung aufweisen. Es hat sich gezeigt, dass dies in der Einbringstellung der Fixierelemente 12 und 13 der Fixiereinrichtung 46 dazu führt, dass die Fixierelemente 12 und 13 mit ihren Spitzen 21 und 22 jeweils verbessert in die Körpergewebe 42 bzw. 43 eindringen können, als dies bei der Fixiereinrichtung 10 der Fall ist. Weiter können die Fixierelemente 12 und 13 aufgrund der jeweiligen konkaven Krümmung der Fixierglieder 18 und 20 tiefer in die Körpergewebe 42 und 43 eindringen, als das bei der Fixiereinrichtung 10 der Fall ist.

Im Übrigen funktioniert die Fixiereinrichtung 46 genauso wie die Fixiereinrichtung 10, so dass diesbezüglich auf die vorstehenden Erläuterungen verwiesen werden kann.

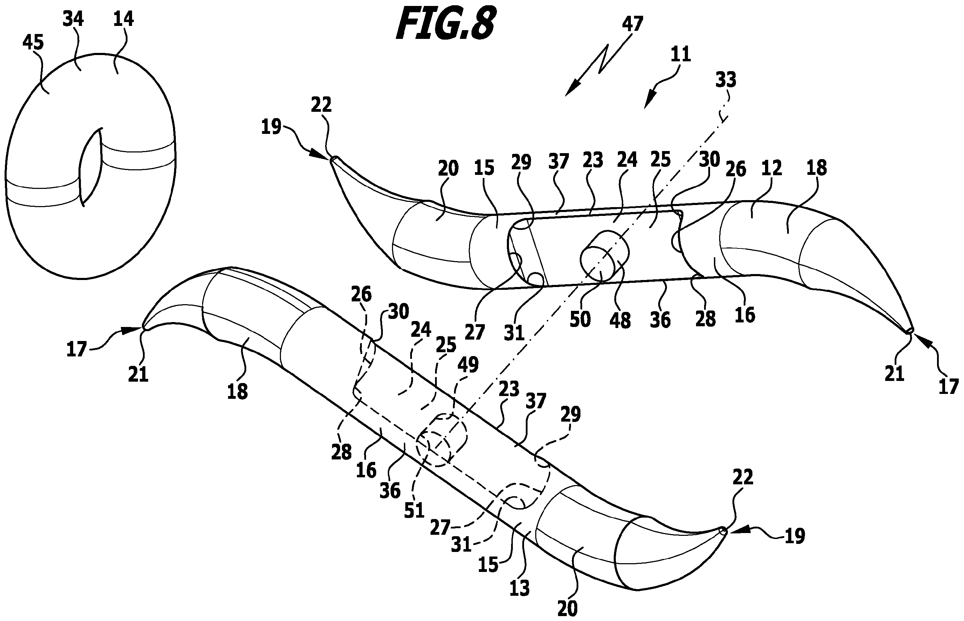

Eine dritte bevorzugte Ausführungsform einer erfindungsgemäßen Fixiereinrichtung ist in Figur 8 in einer der Figur 1B entsprechenden Weise dargestellt und dort insgesamt mit dem Bezugszeichen 47 belegt. Die Fixiereinrichtung 47 ist weitestgehend identisch ausgebildet zur Fixiereinrichtung 10, und für ihre Merkmale werden die identischen Bezugszeichen wie für die Merkmale der Fixiereinrichtung 10 verwendet.

Bei der Fixiereinrichtung 47 umfassen die Fixierelemente 12 und 13 zusammenwirkende Lagerelemente 48 bzw. 49. Das Lagerelement 48 ist am Fixierelement 12 mittig zwischen den Enden 17 und 19 angeordnet und erstreckt sich vom Anlageglied 25, zu dem es senkrecht ausgerichtet ist, in Richtung des Fixierelementes 13. Die Erstreckung des Lagerzapfens 50 definiert den Verlauf der Schwenkachse 33. Das Lagerelement 49 ist eine mittig zwischen den Enden 17 und 19 des Fixierelementes 13 angeordnete Ausnehmung in Form eines Sackloches 51. In das Sackloch 51 kann der Lagerzapfen 50 formschlüssig eingreifen. Die Verschwenkung der Fixierelemente 12 und 13 relativ zueinander kann dadurch auf zuverlässige Weise erfolgen. Gleichzeitig kann eine ungewollte translatorische Bewegung der Fixierelemente 12 und 13 relativ zueinander entlang der durch die Anlageglieder 25 definierten Ebenen vermieden werden. Dies stellt eine noch zuverlässigere Funktion der Fixiereinrichtung 47 sicher.

Anstelle als Lagerzapfen 50 könnte das Lagerelement 48 auch als konvexer Vorsprung ausgebildet sein. Dementsprechend könnte das Lagerelement 49 anstatt als Sackloch 51 als konkave Aufnahme ausgebildet sein und beispielsweise durch eine konkave Prägung gefertigt sein.

Im Übrigen funktioniert die Fixiereinrichtung 47 genauso wie die Fixiereinrichtung 10, so dass diesbezüglich auf die vorstehenden Erläuterungen verwiesen werden kann.

Die Lagerelemente 48 und 49 könnten auch bei der Fixiereinrichtung 46 vorgesehen sein.

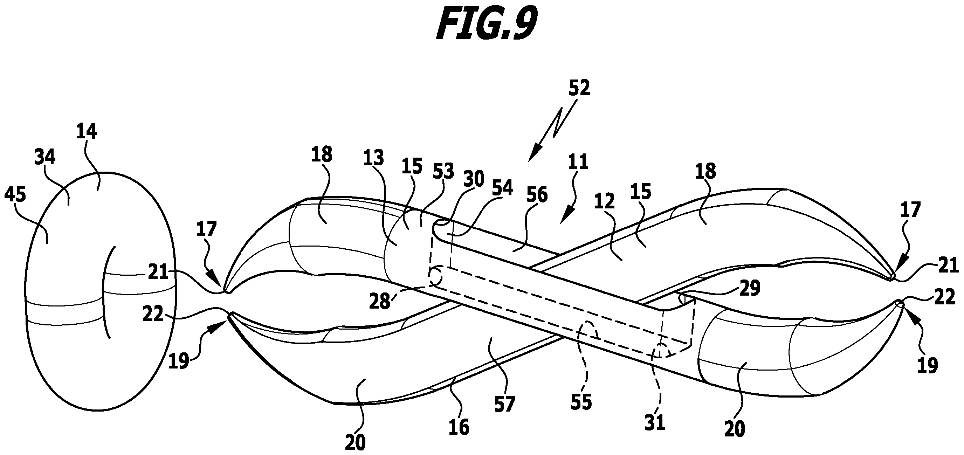

Eine vierte bevorzugte Ausführungsform einer erfindungsgemäßen Fixiereinrichtung ist in Figur 9 teilweise in Explosionsdarstellung dargestellt und dort insgesamt mit dem Bezugszeichen 52 belegt. Merkmale der Fixiereinrichtung 52, die gleich oder gleichwirkend zu Merkmalen der Fixiereinrichtung 10 sind, werden mit identischen Bezugszeichen belegt.

Das Fixierelement 13 der Fixiereinrichtung 52 weist anders als das Fixierelement 13 der Fixiereinrichtung 10 einen längserstreckten geradlinigen mittleren Abschnitt 53 auf. Der mittlere Abschnitt 53 umfasst eine schlitzförmige Durchbrechung 54, die quer zur Erstreckung des Fixierelementes 13 von Seitenwänden 55 und 56 begrenzt ist.

Das Fixierelement 12 der Fixiereinrichtung 52 ist anders als das Fixierelement 12 der Fixiereinrichtung 10 beidseitig jeweils über seine gesamte Länge abgeflacht ausgebildet, wobei es an einer Seite eine planare Seitenfläche 57 aufweist und an der anderen Seite ebenfalls eine planare und in der Zeichnung nicht dargestellte Seitenfläche.

Das Fixierelement 12 durchgreift das Fixierelement 13 an der Durchbrechung 54 formschlüssig, wobei die Seitenwand 55 an der Seitenfläche 57 anliegt und die Seitenwand 56 an der in der Zeichnung nicht dargestellten Seitenfläche. Auf diese Weise wirken die Seitenwände 55 und 56 sowohl als Anlageglieder als auch als Lagerelemente für die Seitenfläche 57 bzw. die nicht dargestellte Seitenfläche und umgekehrt. Auf diese Weise sind die Fixierelemente 12 und 13 gegen eine Bewegung relativ zueinander quer zur Erstreckung der Durchbrechung 54 gesichert. Im Bereich der Überkreuzung der Fixierelemente 12 und 13 ist dadurch ein Kastenschluss gebildet.

Im Übrigen funktioniert die Fixiereinrichtung 52 genauso wie die Fixiereinrichtung 10, so dass diesbezüglich auf die vorstehenden Erläuterungen verwiesen werden kann.

## Patentansprüche

1. Medizinische Fixiereinrichtung zur Fixierung eines ersten Körpergewebes (42) an einem zweiten Körpergewebe (43), umfassend ein erstes Fixierelement (12), ein relativ zu diesem beweglich ausgebildetes zweites Fixierelement (13) sowie ein das erste Fixierelement (12) und das zweite Fixierelement (13) zusammenhaltendes Halteelement (14), bei welcher das erste Fixierelement (12) mindestens ein erstes Fixierglied (18, 20) zum Eindringen in eines der Körpergewebe (42, 43) aufweist und das zweite Fixierelement (13) mindestens ein zweites Fixierglied (18, 20) zum Eindringen in das andere Körpergewebe (42, 43), wobei das erste Fixierelement (12) relativ zum zweiten Fixierelement (13) von einer Einbringstellung in eine Fixierstellung überführbar ist, in welcher Fixierstellung das mindestens eine erste Fixierglied (18, 20) und das mindestens eine zweite Fixierglied (18, 20) einen kleineren Abstand voneinander aufweisen als in der Einbringstellung, und wobei das Halteelement (14) ein Vorspannelement (45) ausbildet zur Vorspannung des ersten Fixierelementes (12) und des zweiten Fixierelementes (13) relativ zueinander in ihrer Einbringstellung bezüglich ihrer Fixierstellung, **dadurch gekennzeichnet, dass** das erste Fixierelement (12) und das zweite Fixierelement (13) einander an einem Überkreuzungsbereich (32) der Fixiereinrichtung (10; 46; 47; 52) überkreuzen, dass das mindestens eine erste Fixierglied (18, 20) und das mindestens eine zweite Fixierglied (18, 20) jeweils einen Abstand zum Überkreuzungsbereich (32) aufweisen und dass das erste Fixierelement (12) und das zweite Fixierelement (13) relativ zueinander um eine durch den Überkreuzungsbereich (32) verlaufende Schwenkachse (33) verschwenkbar ausgebildet sind.

2. Fixiereinrichtung nach Anspruch 1, **dadurch gekennzeichnet, dass** das erste Fixierelement (12) und/oder das zweite Fixierelement (13) längserstreckt ausgebildet ist.

3. Fixiereinrichtung nach Anspruch 1 oder 2, **dadurch gekennzeichnet, dass** das mindestens eine erste Fixierglied (18, 20) endseitig am ersten Fixierelement (12) und/oder dass das mindestens eine zweite Fixierglied (18, 20) endseitig am zweiten Fixierelement (13) angeordnet ist.

4. Fixiereinrichtung nach einem der voranstehenden Ansprüche, **dadurch gekennzeichnet, dass** das erste Fixierelement (12) zwei Fixierglieder (18, 20) umfasst und/oder dass das zweite Fixierelement (13) zwei Fixierglieder (18, 20) umfasst.

5. Fixiereinrichtung nach Anspruch 4, **dadurch gekennzeichnet, dass** die zwei ersten Fixierglieder (18, 20) des ersten Fixierelementes (12) an einander gegenüberliegenden Enden (17, 19) des ersten Fixierelementes (12) angeordnet sind und/oder dass die zwei zweiten Fixierglieder (18, 20) des zweiten Fixierelementes (13) an einander gegenüberliegenden Enden (17, 19) des zweiten Fixierelementes (13) angeordnet sind.

6. Fixiereinrichtung nach einem der voranstehenden Ansprüche, **dadurch gekennzeichnet, dass** das Halteelement (14) das erste Fixierelement (12) und das zweite Fixierelement (13) umgreift.

7. Fixiereinrichtung nach Anspruch 6, **dadurch gekennzeichnet, dass** das Halteelement (14) das erste Fixierelement (12) und das zweite Fixierelement (13) jeweils mittig zwischen zwei Enden (17, 19) des ersten Fixierelementes (12) und zwei Enden (17, 19) des zweiten Fixierelementes (13) umgreift.

8. Fixiereinrichtung nach einem der voranstehenden Ansprüche, **dadurch gekennzeichnet, dass** das Halteelement (14) mindestens ein elastisches Element (34) umfasst oder ausbildet, das in der Einbringstellung des ersten Fixierelementes (12) und des zweiten Fixierelementes (13) einen gespannten Zustand einnimmt und in der Fixierstellung des ersten Fixierelementes (12) und des zweiten Fixierelementes (13) einen entspannteren Zustand einnimmt.

9. Fixiereinrichtung nach einem der voranstehenden Ansprüche, **dadurch gekennzeichnet, dass** das Halteelement (14) als ein das erste Fixierelement (12) und das zweite Fixierelement (13) umgebender O-Ring (34) ausgestaltet ist.

10. Fixiereinrichtung nach einem der voranstehenden Ansprüche, **dadurch gekennzeichnet, dass** das erste Fixierelement (12) ein erstes Anlageglied (25) für das zweite Fixierelement (13) aufweist und dass das zweite Fixierelement (13) ein zweites Anlageglied (25) für das erste Fixierelement (12) aufweist und dass das erste Fixierelement (12) und das zweite Fixierelement (13) über das erste Anlageglied (25) und das zweite Anlageglied (25) aneinander anliegen.

11. Fixiereinrichtung nach Anspruch 10, **dadurch gekennzeichnet, dass** das erste Anlageglied (25) und/oder das zweite Anlageglied (25) planar ausgestaltet ist.

12. Fixiereinrichtung nach einem der voranstehenden Ansprüche, **dadurch gekennzeichnet, dass** das erste Fixierelement (12) ein erstes Lagerelement (25; 48; 57) umfasst, das mit einem vom zweiten Fixierelement (13) umfassten zweiten Lagerelement (25; 49; 55; 56) zur Lagerung des ersten Fixierelementes (12) und des zweiten Fixierelementes (13) aneinander zusammenwirkt.

13. Fixiereinrichtung nach einem der voranstehenden Ansprüche, **dadurch gekennzeichnet, dass** ein Fixierelement (12, 13) eine in dem anderen Fixierelement (12, 13) gebildete Durchbrechung (54) durchgreift.

14. Fixiereinrichtung nach einem der voranstehenden Ansprüche, **dadurch gekennzeichnet, dass** das erste Fixierelement (12) und/oder das zweite Fixierelement (13) und/oder das Halteelement (14) einstückig ausgebildet ist.

15. Fixiereinrichtung nach einem der voranstehenden Ansprüche, **dadurch gekennzeichnet, dass** mindestens ein Fixierelement (12, 13) in sich symmetrisch ausgebildet ist.

## Claims

1. Medical fixing device for fixing a first body tissue (42) to a second body tissue (43), comprising a first fixing element (12), a second fixing element (13) constructed so as to be movable relative thereto, and a holding element (14) holding the first fixing element (12) and the second fixing element (13) together, in which the first fixing element (12) comprises at least one first fixing member (18, 20) for penetration into one of the body tissues (42, 43) and the second fixing element (13) comprises at least one second fixing member (18, 20) for penetration into the other body tissue (42, 43), the first fixing element (12) being transferrable relative to the second fixing element (13) from an insertion position to a fixing position, in which fixing position the at least one first fixing member (18, 20) and the at least one second fixing member (18, 20) are at a smaller distance from each other than in the insertion position, and the holding element (14) forming a pretensioning element (45) for pretensioning the first fixing element (12) and the second fixing element (13) relative to each other in their insertion position with respect to their fixing position, **characterized in that** the first fixing element (12) and the second fixing element (13) intersect at an intersection area (32) of the fixing device (10; 46; 47; 52), **in that** the at least one first fixing member (18, 20) and the at least one second fixing member (18, 20) are each at a distance from the intersection area (32), and **in that** the first fixing element (12) and the second fixing element (13) are constructed for swivel movement relative to each other about a swivel axis (33) running through the intersection area (32).

2. Fixing device in accordance with claim 1, **characterized in that** the first fixing element (12) and/or the second fixing element (13) is/are of elongate construction.

3. Fixing device in accordance with claim 1 or 2, **characterized in that** the at least one first fixing member (18, 20) is arranged at the end of the first fixing element (12) and/or **in that** the at least one second fixing member (18, 20) is arranged at the end of the second fixing element (13).

4. Fixing device in accordance with any one of the preceding claims, **characterized in that** the first fixing element (12) comprises two fixing members (18, 20) and/or **in that** the second fixing element (13) comprises two fixing members (18, 20).

5. Fixing device in accordance with claim 4, **characterized in that** the two first fixing members (18, 20) of the first fixing element (12) are arranged at opposite ends (17, 19) of the first fixing element (12) and/or **in that** the two second fixing members (18, 20) of the second fixing element (13) are arranged at opposite ends (17, 19) of the second fixing element (13).

6. Fixing device in accordance with any one of the preceding claims, **characterized in that** the holding element (14) engages around the first fixing element (12) and the second fixing element (13).

7. Fixing device in accordance with claim 6, **characterized in that** the holding element (14) engages around the first fixing element (12) and the second fixing element (13), in each case, midway between two ends (17, 19) of the first fixing element (12) and two ends (17, 19) of the second fixing element (13).

8. Fixing device in accordance with any one of the preceding claims, **characterized in that** the holding element (14) comprises or forms at least one elastic element (34) which, in the insertion position of the first fixing element (12) and the second fixing element (13), assumes a taut state and, in the fixing position of the first fixing element (12) and the second fixing element (13), assumes a more relaxed state.

9. Fixing device in accordance with any one of the preceding claims, **characterized in that** the holding element (14) is configured as an O-ring (34) surrounding the first fixing element (12) and the second fixing element (13).

10. Fixing device in accordance with any one of the preceding claims, **characterized in that** the first fixing element (12) comprises a first abutment member (25) for the second fixing element (13), and **in that** the second fixing element (13) comprises a second abutment member (25) for the first fixing element (12), and **in that** the first fixing element (12) and the second fixing element (13) abut against each other by way of the first abutment member (25) and the second abutment member (25).

11. Fixing device in accordance with claim 10, **characterized in that** the first abutment member (25) and/or the second abutment member (25) is/are of planar configuration.

12. Fixing device in accordance with any one of the preceding claims, **characterized in that** the first fixing element (12) comprises a first mounting element (25; 48; 57) which interacts with a second mounting element (25; 49; 55; 56) comprised by the second fixing element (13) for mounting the first fixing element (12) and the second fixing element (13) on each other.

13. Fixing device in accordance with any one of the preceding claims, **characterized in that** one fixing element (12, 13) extends through an opening (54) formed in the other fixing element (12, 13).

14. Fixing device in accordance with any one of the preceding claims, **characterized in that** the first fixing element (12) and/or the second fixing element (13) and/or the holding element (14) is/are of one piece construction.

15. Fixing device in accordance with any one of the preceding claims, **characterized in that** at least one fixing element (12, 13) is of inherently symmetrical construction.

## Revendications

1. Dispositif de fixation médical pour fixer un premier tissu corporel (42) à un deuxième tissu corporel (43), qui comprend un premier élément de fixation (12), un deuxième élément de fixation (13) réalisé relativement mobile par rapport au premier, ainsi qu'un élément de maintien (14) qui maintient assemblé le premier élément de fixation (12) et le deuxième élément de fixation (13), dispositif
dans lequel le premier élément de fixation (12) présente au moins un premier organe de fixation (18, 20) destiné à pénétrer dans l'un des tissus corporels (42, 43), et le deuxième élément de fixation (13) présente au moins un deuxième organe de fixation (18, 20) destiné à pénétrer dans l'autre tissu corporel (42, 43),
dans lequel le premier élément de fixation (12) peut être transféré par rapport au deuxième élément de fixation (13) d'une position de mise en place à une position de fixation, position de fixation dans laquelle ledit au moins un premier organe de fixation (18, 20) et ledit au moins un deuxième organe de fixation (18, 20) présentent une distance d'espacement réciproque plus petite que dans la position de mise en place,
et dans lequel l'élément de maintien (14) forme un élément de précontrainte (45) pour assurer la précontrainte du premier élément de fixation (12) et du deuxième élément de fixation (13) l'un par rapport à l'autre dans leur position de mise en place par rapport à leur position de fixation,
**caractérisé en ce que** le premier élément de fixation (12) et le deuxième élément de fixation (13) se croisent mutuellement au niveau d'une zone de croisement (32) du dispositif de fixation (10; 46 ; 47; 52), **en ce que** ledit au moins un premier organe de fixation (18, 20) et ledit au moins un deuxième organe de fixation (18, 20) présentent respectivement une distance d'espacement de la zone de croisement (32), et **en ce que** le premier élément de fixation (12) et le deuxième élément de fixation (13) sont réalisés de manière à pouvoir pivoter l'un par rapport à l'autre autour d'un axe de pivotement (33) s'étendant à travers la zone de croisement (32).

2. Dispositif de fixation selon la revendication 1,
**caractérisé en ce que** le premier élément de fixation (12) et/ou le deuxième élément de fixation (13) sont d'une configuration allongée.

3. Dispositif de fixation selon la revendication 1 ou la revendication 2, **caractérisé en ce que** ledit au moins un premier organe de fixation (18, 20) est agencé en extrémité sur le premier élément de fixation (12), et/ou ledit au moins un deuxième organe de fixation (18, 20) est agencé en extrémité sur le deuxième élément de fixation (13).

4. Dispositif de fixation selon l'une des revendications précédentes, **caractérisé en ce que** le premier élément de fixation (12) comporte deux organes de fixation (18, 20), et/ou le deuxième élément de fixation (13) comporte deux organes de fixation (18, 20).

5. Dispositif de fixation selon la revendication 4,
**caractérisé en ce que** les deux premiers organes de fixation (18, 20) du premier élément de fixation (12) sont agencés à des extrémités (17, 19) mutuellement opposées du premier élément de fixation (12), et/ou **en ce que** les deux deuxièmes organes de fixation (18, 20) du deuxième élément de fixation (13) sont agencés à des extrémités (17, 19) mutuellement opposées du deuxième élément de fixation (13).

6. Dispositif de fixation selon l'une des revendications précédentes, **caractérisé en ce que** l'élément de maintien (14) entoure le premier élément de fixation (12) et le deuxième élément de fixation (13).

7. Dispositif de fixation selon la revendication 6,
**caractérisé en ce que** l'élément de maintien (14) entoure le premier élément de fixation (12) et le deuxième élément de fixation (13) chacun respectivement en son milieu, entre deux extrémités (17, 19) du premier élément de fixation (12) et deux extrémités (17, 19) du deuxième élément de fixation (13).

8. Dispositif de fixation selon l'une des revendications précédentes, **caractérisé en ce que** l'élément de maintien (14) comprend ou forme au moins un élément élastique (34), qui, dans la position de mise en place du premier élément de fixation (12) et du deuxième élément de fixation (13) prend un état en tension, et, dans la position de fixation du premier élément de fixation (12) et du deuxième élément de fixation (13), prend un état plus détendu.

9. Dispositif de fixation selon l'une des revendications précédentes, **caractérisé en ce que** l'élément de maintien (14) est réalisé sous forme de joint torique (34) entourant le premier élément de fixation (12) et le deuxième élément de fixation (13).

10. Dispositif de fixation selon l'une des revendications précédentes, **caractérisé en ce que** le premier élément de fixation (12) présente un premier organe d'appui (25) pour le deuxième élément de fixation (13), et **en ce que** le deuxième élément de fixation (13) présente un deuxième élément d'appui (25) pour le premier élément de fixation (12), et **en ce que** le premier élément de fixation (12) et le deuxième élément de fixation (13) s'appuient l'un sur l'autre par l'intermédiaire du premier organe d'appui (25) et du deuxième organe d'appui (25).

11. Dispositif de fixation selon la revendication 10,
**caractérisé en ce que** le premier organe d'appui (25) et/ou le deuxième organe d'appui (25) sont de configuration planaire.

12. Dispositif de fixation selon l'une des revendications précédentes, **caractérisé en ce que** le premier élément de fixation (12) comprend un premier élément de palier (25; 48; 57), qui interagit avec un deuxième élément de palier (25; 49; 55; 56) que comprend le deuxième élément de fixation (13), pour assurer le montage du premier élément de fixation (12) et du deuxième élément de fixation (13) l'un avec l'autre.

13. Dispositif de fixation selon l'une des revendications précédentes, **caractérisé en ce qu'**un élément de fixation (12, 13) traverse un passage traversant (54) formé dans l'autre élément de fixation (12, 13).

14. Dispositif de fixation selon l'une des revendications précédentes, **caractérisé en ce que** le premier élément de fixation (12) et/ou le deuxième élément de fixation (13) et/ou l'élément de maintien (14) sont réalisés d'un seul tenant.

15. Dispositif de fixation selon l'une des revendications précédentes, **caractérisé en ce qu'**au moins un élément de fixation (12, 13) est réalisé symétrique en soi.
